# EUROPEAN PATENT APPLICATION

(11) **EP 4 678 126 A1**
(43) Date of publication of application: **14.01.2026**
(21) Application number: 25188004.3
(22) Date of filing: 08.07.2025
(51) Int. Cl.: A61B 18/14, A61B 90/98

(54) **DERMATOLOGIC TREATMENT DEVICE WITH NFC CHIP AND OPERATING METHOD OF THE SAME**

(30) Priority: 08.07.2024 KR 20240089430
(71) Applicant: VIOL Co. Ltd., Bundang-gu Seongnam-si, Gyeonggi-do, 13510 (KR)
(72) Inventor: LEE, Sangjin, 13510 Seongnam-si, Gyeonggi-do (KR); KIM, Jongkil, 13510 Seongnam-si, Gyeonggi-do (KR); JEON, Yeonggi, 13510 Seongnam-si, Gyeonggi-do (KR); LEE, Jaebeom, 13510 Seongnam-si, Gyeonggi-do (KR)
(74) Representative: Stöckeler, Ferdinand

(57) **Abstract**

Disclosed is a dermatologic treatment device performing a skin procedure operation. The dermatologic treatment device includes a treatment tip configured to come into contact with the skin of a patient and to transmit energy and an applicator connected to the treatment tip and configured to control the treatment tip. The treatment tip includes a near field communication (NFC) chip configured to store treatment tip data. The applicator is configured to read the treatment tip data from the NFC chip and to perform the skin procedure operation based on the treatment tip data.

## Description

### [Technical Field]

The present disclosure relates to a dermatologic treatment device and an operating method thereof and, more particularly, to a dermatologic treatment device including a near field communication (NFC) chip, which can perform wireless communication with the outside in a contactless manner, and an operating method thereof.

### [Background Art]

Recently, various procedures for skin care and obesity treatment are being developed. Among the various procedures, an invasive dermatologic treatment device includes a needle configured to penetrate a skin tissue, and applies radio frequency (RF) power or heat to the skin tissue so that the skin tissue is treated and improved when the needle penetrates the skin tissue. A non-invasive dermatologic treatment device does not directly penetrate the skin, and applies RF power or heat to the skin so that a skin tissue is treated and improved in the state in which the non-invasive dermatologic treatment device has come into contact with the skin.

Due to the nature of a dermatologic treatment device that performs a procedure by penetrating or coming into contact with the skin, a treatment tip that comes into direct contact with the skin is manufactured separately from a handpiece, that is, a handle part of the treatment device, and manufactured and supplied as a consumable product that is intended for single use or to be replaced and consumed after a predetermined number of uses.

In a conventional technology, there is a problem in that such a treatment tip is not separately managed. However, as the sales scale of the treatment tip is increased, there is a need for a method of individually managing the treatment tip because the treatment tip needs to be managed from a manufacturer's point of view and there is an increasing need for a treatment tip and procedure method suitable for the characteristics of a patient who experiences a procedure from the patient's point of view.

### [Summary of Invention]

### [Technical Problem]

Embodiments of the present disclosure provide a dermatologic treatment device including an NFC chip and an operating method thereof, which can perform wireless communication with the outside in a contactless manner.

### [Solution to Problem]

In an embodiment, a dermatologic treatment device performing a skin procedure operation may include a treatment tip configured to come into contact with the skin of a patient and to deliver energy and an applicator connected to the treatment tip and configured to control the treatment tip. The treatment tip includes a near field communication (NFC) chip configured to store treatment tip data. The applicator is configured to read the treatment tip data from the NFC chip and to perform the skin procedure operation based on the treatment tip data.

In an embodiment, an operating method of a dermatologic treatment device including a treatment tip equipped with a near field communication (NFC) chip may include detecting the connection of the treatment tip, reading treatment tip data from the NFC chip of the connected treatment tip when the treatment tip is connected, and performing a skin procedure operation based on the treatment tip data.

### [Advantageous Effects of Invention]

The dermatologic treatment device according to embodiments of the present disclosure can be usefully used in the inventory management, history management, and shipment management of the treatment tip because the dermatologic treatment device includes the treatment tip equipped with the NFC chip.

In particular, from a treatment tip manufacturer's point of view, there is an effect in that the history and inventory management of a treatment tip product is further facilitated because a process of producing, distributing, and selling the treatment tip product can be tracked and managed and a movement and quality of each treatment tip product can be tracked by assigning a unique identifier to each treatment tip product. Moreover, it is possible to certify a genuine product of a treatment tip product and to prevent the duplication of the treatment tip product because digital signature is included in the treatment tip product.

Furthermore, the treatment tip can be identified through a separate NFC terminal even without releasing the packaging of the specifications of the treatment tip in an individually packaged state. Accordingly, it is possible to improve use convenience and efficiency because the specifications of the treatment tip can be checked easily and simply.

Furthermore, the procedure stability and effect of a patient can be increased because the applicator reads data stored in the treatment tip when the treatment tip is mounted on the applicator upon procedure and operates based on the read data.

### [Brief Description of Drawings]

FIG. 1 illustrates a dermatologic treatment device according to embodiments of the present disclosure.
FIG. 2 is a flowchart illustrating an operating method of the applicator according to embodiments of the present disclosure.
FIG. 3 is a flowchart illustrating an operating method of the applicator according to embodiments of the present disclosure.
FIG. 4 is a flowchart illustrating an operating method of the applicator according to embodiments of the present disclosure.
FIG. 5 illustrates a form in which a treatment tip and a handpiece according to embodiments of the present disclosure are mounted.
FIG. 6 illustrates a form in which the treatment tip and the handpiece according to embodiments of the present disclosure are separated from each other.
FIG. 7 is a separated perspective view of a treatment tip according to embodiments of the present disclosure.
FIG. 8 is a cross-sectional view illustrating a cross-sectional structure of the treatment tip according to embodiments of the present disclosure.
FIG. 9 illustrates a form in which a near field communication (NFC) chip has been installed in a first substrate of the treatment tip.
FIGS. 10 and 11 illustrate plan and rear structures of the first substrate illustrated in FIG. 9.
FIG. 12 illustrates a front end part structure of the handpiece in which a plurality of RF supply terminals and signal transmission terminals connected to a plurality of RF input electrodes and signal transmission electrodes formed in the first substrate, respectively, are arranged.

### [Description of Embodiments]

Hereinafter, embodiments of the present disclosure are described in detail with reference to the accompanying drawings.

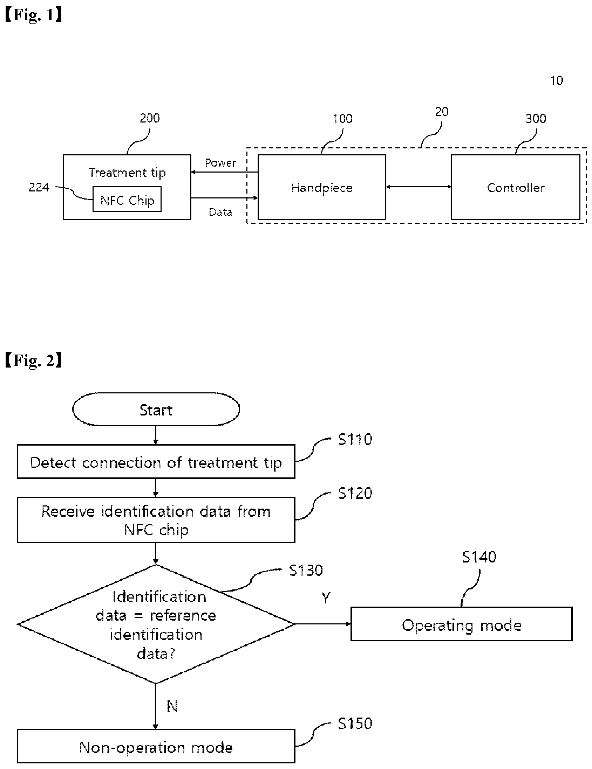

FIG. 1 illustrates a dermatologic treatment device according to embodiments of the present disclosure. A dermatologic treatment device 10 may include a handpiece 100, a treatment tip 200 that is detachably combined with the handpiece 100, and a controller 300 configured to control the handpiece 100.

The treatment tip 200 is mounted at the front end of the handpiece 100 and detachably mounted on the handpiece 100 in a replaceable cartridge manner.

Parts for a skin procedure may be mounted on the treatment tip 200. For example, in the case of an invasive dermatologic treatment device, a plurality of micro needles may be mounted on the treatment tip 200. For example, in the case of a non-invasive dermatologic treatment device, a transducer or an RF electrode may be mounted on the treatment tip 200.

The handpiece 100 may be connected to the controller 300 in a wired or wireless manner, and may be combined with the treatment tip 200 mechanically or electrically.

The handpiece 100 may control an operation of the treatment tip 200. According to embodiments, the handpiece 100 may control a relative movement of the treatment tip 200 for the handpiece 100. For example, when the treatment tip 200 is a needle tip in which micro needles are disposed, the handpiece 100 may include a driving unit for driving forward and backward movements of the micro needles. The driving unit may move a circuit board on which the micro needles included in the needle tip 200 are mounted forward or backward within a predetermined stroke range.

The controller 300 may control an overall operation of the handpiece 100. According to embodiments, the controller 300 may supply power for a skin treatment procedure to the handpiece 100. For example, if a skin treatment procedure is based on radio frequency (RF) power, the controller 300 may generate RF power and transmit the RF power to the handpiece 100, or may generate power necessary for RF power and transmit the power to the handpiece 100. Furthermore, for example, if a skin treatment procedure is based on ultrasonic waves, the controller 300 may generate power for an operation of an ultrasonic transducer and transmit the power to the handpiece 100.

The treatment tip 200 is replaced and used as a replacement every procedure or at a predetermined cycle. The handpiece 100 and the controller 300 may be consistently used regardless of the replacement of a treatment tip. In this viewpoint, the handpiece 100 and the controller 300 are collectively called an applicator 20. That is, the applicator 20 may be understood as a device that is combined with the treatment tip 200 and that induces an operation of the treatment tip 200 and performs a series of control operations for performing a skin treatment operation through the treatment tip 200. Hereinafter, an operation of the applicator 20 described in this specification is understood as an operation of the handpiece 100 and/or the controller 300.

The dermatologic treatment device according to embodiments of the present disclosure includes the treatment tip 200 including a near field communication (NFC) chip 224, and may operate based on treatment tip data included in the NFC chip 224 of the treatment tip 200.

The treatment tip data may include data related to an operation of the dermatologic treatment device. According to embodiments, the treatment tip data may include at least one of identification data for identifying whether the treatment tip 200 can be used normally, count data indicative of the number of uses of the treatment tip 200, and operation data indicative of an operation environment for the treatment tip 200, but the present disclosure is not limited thereto.

The treatment tip data may be written in a wireless manner through an external NFC terminal. For example, after the production and packaging of the treatment tip 200 are completed, predetermined information may be written as the treatment tip data through an NFC writer. Accordingly, although the sealing of the treatment tip 200 is not released, it is possible to write the treatment tip data in the treatment tip 200.

According to embodiments, the NFC chip 224 may include an antenna, NFC storage in which treatment tip data have been stored, and an NFC controller capable of accessing the NFC storage. The NFC controller may write treatment tip data stored in the NFC storage based on a read/write command that is transmitted from the outside or may write treatment tip data in the NFC storage. The read/write command may be wirelessly received through the antenna included in the NFC chip 224, or may be input to the NFC controller in a wired signal manner through an electrical path.

When the treatment tip 200 is mounted on the applicator 20, the applicator 20 may supply predetermined power to the NFC chip 224 of the treatment tip 200 and read treatment tip data stored in the NFC chip 224. According to embodiments, the applicator 20 may transmit, to the NFC controller included in the NFC chip 224, a command that instructs the reading of treatment tip data stored in the NFC storage accessible to the NFC controller. In response to the command, the NFC controller may transmit the treatment tip data to the applicator 20. For example, the controller 300 may generate a command for reading treatment tip data stored in the NFC storage and directly output the command to the NFC chip 224 or may output the command to the NFC chip 224 through the handpiece 100, but the present disclosure is not limited thereto.

According to embodiments, a data path for the communication of treatment tip data may be formed between the handpiece 100 and the NFC chip 224 of the treatment tip 200. Treatment tip data stored in the NFC chip 224 may be transmitted from the NFC chip 224 to the handpiece 100 through the data path. For example, the data path may be a data path using an inter integrated circuit (I2C) method, but the present disclosure is not limited thereto. Furthermore, the treatment tip data received by the handpiece 100 may be transmitted to the controller 300.

When the applicator 20 reads treatment tip data stored in the NFC chip 224, the applicator 20 may operate based on the read treatment tip data. For example, the applicator 20 may compare treatment tip data stored in the NFC chip 224 and reference treatment tip data stored in the applicator 20, and may operate based on a result of the comparison. Alternatively, the applicator 20 may adjust an operation variable of the applicator 20 based on treatment tip data stored in the NFC chip 224 and operate based on the adjusted operation variable, and a detailed operation thereof is described later.

According to embodiments of the present disclosure, information related to the treatment tip 200 can be written and stored in the NFC chip 224 within the treatment tip 200 in a contactless manner. Accordingly, treatment tip data can be easily written en bloc after the production and packaging of the treatment tip 200. Furthermore, the treatment tip data may be used to control an operation of the dermatologic treatment device upon subsequent procedure.

FIG. 2 is a flowchart illustrating an operating method of the applicator according to embodiments of the present disclosure.

Referring to FIG. 2, the applicator 20 detects the connection of the treatment tip 200 (S110). According to embodiments, the applicator 20 may detect the connection of the treatment tip 200 through an electrical or physical method. For example, the handpiece 100 may include predetermined detection means. The detection means may detect the connection of the treatment tip 200 physically or electrically, and may transmit an electrical signal to the controller 300.

According to embodiments, the applicator 20 may detect the connection of the treatment tip 200 whenever the treatment tip 200 is newly connected.

The applicator 20 receives identification data from the NFC chip 224 (S120). According to embodiments, the identification data are data for identifying whether the treatment tip 200 can be used normally. For example, the identification data may be data indicative of a country in which the treatment tip 200 is used, whether the treatment tip 200 is a genuine product, or the serial number of the treatment tip 200.

The applicator 20 may compare the read identification data and pre-stored reference identification data (S130). According to embodiments, the comparison of the identification data and the reference identification data may be performed by the handpiece 100 or the controller 300. The reference identification data may be stored in the applicator 20 in advance.

For example, when the identification data indicate the country in which the treatment tip 200 is used, the reference identification data may indicate a country in which the applicator 20 is used. Furthermore, for example, when the identification data are data indicative of whether the treatment tip 200 is a genuine product, the reference identification data may be data indicative of the genuine product. Furthermore, for example, when the identification data are data indicative of the serial number of the treatment tip 200, the reference identification data may be data indicative of the serial numbers of treatment tips 200 that are generated normally.

When the identification data and the reference identification data are the same (or when the identification data are included in the reference identification data) (Y in S130) as a result of the comparison (S130), the applicator 20 may operate in an operation mode (S140). The operation mode may be a mode in which the applicator 20 may perform a skin treatment procedure based on a predetermined input. That is, in the operation mode, the applicator 20 may operate normally based on a user's manipulation in the state in which the treatment tip 200 has been connected.

For example, when the identification data indicate "Republic of Korea" and the reference identification data indicate "Republic of Korea, the United States", the applicator 20 may operate in the operation mode, and a user may perform a skin treatment procedure normally by using the applicator 20.

In contrast, when the identification data and the reference identification data are different (or when the identification data are not included in the reference identification data) (N in S130) as a result of the comparison (S130), the applicator 20 may operate in a non-operation mode (S150). The non-operation mode may be a mode in which the applicator 20 does not perform a skin treatment procedure despite a predetermined input. That is, in the non-operation mode, an operation of the applicator 20 is stopped although a user's manipulation is present while the treatment tip 200 is connected. For example, the non-operation mode may be understood as an interrupt or idle mode.

According to embodiments, after the connection of a specific treatment tip 200, when the applicator 20 enters the non-operation mode, the applicator 20 may release the non-operation mode and switch to the operation mode in response to the separation of the specific treatment tip 200 or a predetermined input signal.

For example, when the identification data indicate "Republic of Korea" and the reference identification data indicate "Japan", the applicator 20 may operate in the non-operation mode.

According to embodiments of the present disclosure, the applicator 20 of the dermatologic treatment device 10 operates normally only when information of the treatment tip 200 connected thereto is matched with information previously stored in the applicator 20. Accordingly, there is an effect in that the procedure stability of a patient can be enhanced because the use of the treatment tip 200 which cannot be used when the treatment tip 200 is connected is prevented.

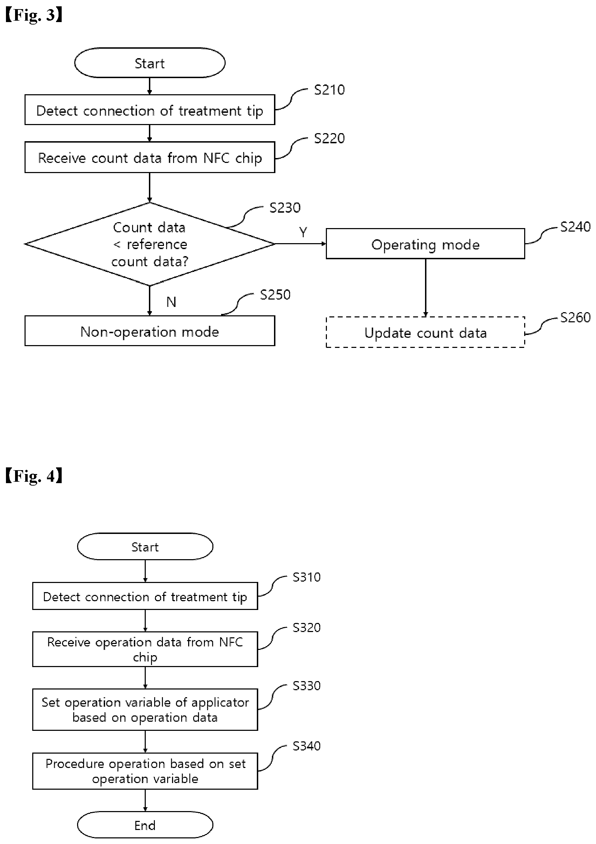

FIG. 3 is a flowchart illustrating an operating method of the applicator according to embodiments of the present disclosure. In this case, a description of the same operation as that of FIG. 2 is omitted.

Referring to FIG. 3, the applicator 20 detects the connection of the treatment tip 200 (S210).

The applicator 20 receives count data from the NFC chip 224 (S220). According to embodiments, the count data are data indicative of the number of uses of the treatment tip 200. For example, when the treatment tip 200 is connected to the applicator 20 and used once, the count data may indicate 1. In contrast, when the treatment tip 200 is connected to the applicator 20 and never used, the count data may indicate 0.

The applicator 20 may compare the read count data and pre-stored reference count data (S230). According to embodiments, the comparison of the count data and the reference count data may be performed by the handpiece 100 or the controller 300. The reference count data may be stored in the applicator 20 in advance.

For example, if the treatment tip 200 cannot be reused, the reference count data may indicate a value (e.g., "1" or "0") indicating that the treatment tip 200 is not used. In contrast, if the treatment tip 200 can be reused, the reference count data may indicate a value (e.g., "200") indicating the use limit of the treatment tip 200, but the present disclosure is not limited thereto.

When the count data are less than the reference identification data (Y in S230) as a result of the comparison (S230), the applicator 20 may operate in the operation mode (S240). In contrast, when the count data are equal to or greater than the reference count data (N in S230) as a result of the comparison (S230), the applicator 20 may operate in the non-operation mode (S250).

According to embodiments, after the treatment tip 200 is connected and a skin procedure is performed, the applicator 20 may write the number of uses of the treatment tip 200 in the NFC chip 224 of the treatment tip 200 whenever the skin procedure is terminated (S260). For example, the applicator 20 may transmit a command indicative of the update of count data or (updated) count data indicative of a corresponding skin procedure number to the NFC chip 224 according to each skin procedure so that the actual number of uses of the treatment tip 200 is written.

According to embodiments of the present disclosure, the applicator 20 of the dermatologic treatment device 10 operates normally only when the number of uses (i.e., count data) of the treatment tip 200 connected thereto is less than a pre-designated recommendation use number (i.e., reference count data). Accordingly, it is possible to enhance the procedure stability of a patient by preventing the reuse of the treatment tip 200.

FIG. 4 is a flowchart illustrating an operating method of the applicator according to embodiments of the present disclosure.

Referring to FIG. 4, the applicator 20 detects the connection of the treatment tip 200 (S310).

The applicator 20 receives operation data from the NFC chip 224 (S320). According to embodiments, the operation data are data indicative of an operation environment for the treatment tip 200. For example, the operation data may include data related to energy (e.g., an RF, heat, or ultrasonic waves) that are transmitted through the treatment tip 200 or data related to movements (e.g., forward and backward movements) during the procedure of the treatment tip 200, the present disclosure is not limited thereto.

For example, the operation data may include data related to the intensity or frequency of RF power when the treatment tip 200 is used. Furthermore, the operation data may include data related to a maximum forward movement distance of the treatment tip 200.

The applicator 20 may set an operation variable of the applicator 20 based on the operation data (S330). According to embodiments, the applicator 20 may determine an operation variable corresponding to an operation environment indicated by the operation data, and may store the operation variable as a setting value.

For example, when the operation data indicate the frequency (e.g., 400 kHz) of RF power when the treatment tip 200 is used, the applicator 20 may set the frequency value of RF power that is used when a procedure is performed as a value (e.g., 400 kHz) corresponding to the operation data with reference to the operation data.

According to embodiments, the applicator 20 may include an interface (e.g., a button or a touch panel) for changing an operation variable. In this case, when an operation variable according to operation data is set after the connection of a specific treatment tip 200, the applicator 20 may reject a change in the operation variable through the interface. However, when a separate external input (e.g., a user mode entry) different from a common change through the interface is received, the applicator 20 may limitedly provide a user with an environment for changing the operation variable.

The applicator 20 may perform a procedure operation based on the set operation variable (S340). According to embodiments, the applicator 20 may transmit energy to the treatment tip 200 based on the set operation variable or may move the treatment tip 200 based on the set operation variable.

According to embodiments of the present disclosure, the applicator 20 of the dermatologic treatment device 10 has an effect in that it can enhance the procedure effect of a patient by automatically setting an operation variable suitable for an operation environment for the treatment tip 200 connected thereto and performing a procedure operation. A patient has only to select the treatment tip 200 having a desired procedure environment. A user has only to connect a corresponding treatment tip 200 and perform a procedure. Accordingly, there is an effect in that an operation environment most suitable for the treatment tip 200 can be automatically set although a separate artificial manipulation is not present.

As described above, the dermatologic treatment device according to embodiments of the present disclosure can be usefully used in the inventory management, history management, and shipment management of the treatment tip 200 because the dermatologic treatment device includes the treatment tip 200 equipped with the NFC chip.

In particular, from the point of view of a manufacturer of the treatment tip 200, the treatment tip 200 may be used to track and manage a process of producing, distributing, and selling a treatment tip product and the movement and quality of each treatment tip product can be tracked by assigning a unique identifier to each treatment tip product. Accordingly, the history and inventory management of the treatment tip product is further facilitated. Moreover, it is possible to authenticate the product as genuine and prevent unauthorized duplication because a digital signature is included in the treatment tip 200.

Furthermore, the treatment tip 200 can be identified through a separate NFC terminal even without releasing the packaging the specifications of the treatment tip 200 in an individually packaged state. Accordingly, use convenience and efficiency can be improved because the specifications of the treatment tip 200 can be checked easily and simply.

Furthermore, when the treatment tip 200 is mounted on the applicator 20 upon procedure, the applicator 20 reads data stored in the treatment tip 200 and operates based on the data. Accordingly, it is possible to enhance the procedure stability and effect of a patient.

Embodiments of the present disclosure are not limited to only a treatment tip having a specific form, but the treatment tip 200 that is used in an invasive medical device, among various types of medical tips, is used as an example hereinafter.

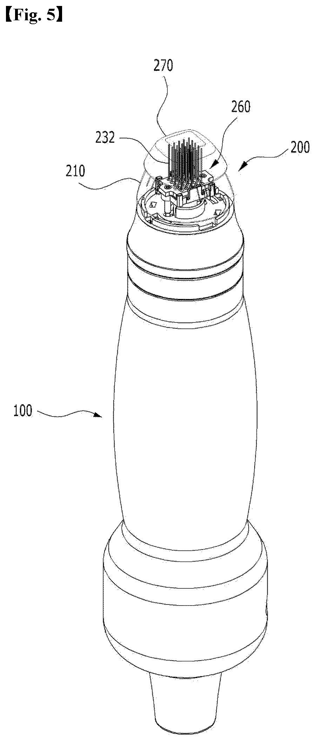

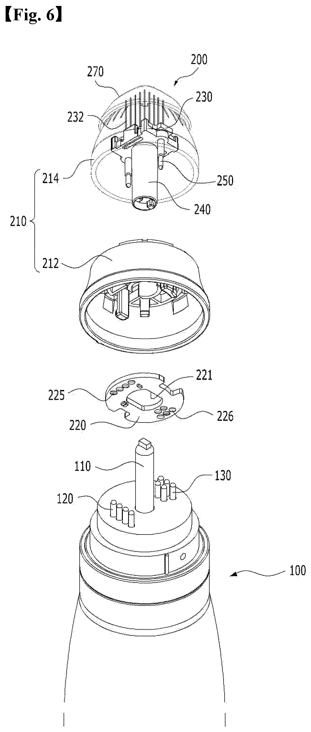

FIG. 5 illustrates a form in which the treatment tip and the handpiece according to embodiments of the present disclosure are mounted. FIG. 6 illustrates a form in which the treatment tip and the handpiece according to embodiments of the present disclosure are separated from each other.

Referring to FIGS. 5 and 6, the treatment tip 200 according to embodiments of the present disclosure is mounted at the front end (i.e., the top of the handpiece in FIG. 5) of the handpiece 100. The treatment tip 200 is detachably mounted at the front end of the handpiece 100 in a cartridge manner so that the treatment tip 200 can be replaced, if necessary.

The treatment tip 200 includes a plurality of micro needles 232 to which an RF current transmitted from the handpiece 100 is applied. The micro needle 232 included in the treatment tip 200 penetrates a target skin tissue of a procedure subject and transfers RF heat energy to the target skin tissue.

The treatment tip 200 may be provided in various types in which the number and arrangement form of the micro needles 232 are different depending on its use. A user may select the treatment tip 200 suitable for a procedure, and may perform a procedure task after mounting the treatment tip 200 on the handpiece 100.

Although not illustrated in the drawings, the handpiece 100 includes a driving unit that drives the micro needle 232 within the treatment tip 200 so that the micro needle 232 reciprocates in a straight line, an RF generator that generates RF transferred to the micro needle 232, and a control unit that controls the driving unit and the RF generator.

The driving unit may move a needle assembly 260 on which the micro needle 232 is mounted in forward and backward directions (i.e., up and down directions in FIG. 5) within a predetermined stroke range. The RF generator may transmit power supplied from the body of the dermatologic treatment device to the micro needle 232 by converting the power in the form of an RF current pulse. The driving unit having various forms, such as a motor, a ball screw, a pneumatic actuator, and an electromagnet actuator, may be applied.

The control unit may be connected to various manipulation buttons included in an external surface of the handpiece 100, and may control operation of the driving unit and the RF generator by generating a control signal according to a manipulation of a manipulation button by a user.

As illustrated in FIG. 6, a part of a driving shaft 110 that is connected to the driving unit of the handpiece 100 is exposed to the outside of the front end of the handpiece 100. The driving shaft 110 exposed to the front end of the handpiece 100 is combined with a holder 240 of the needle assembly 260 included within the treatment tip 200. Accordingly, when the driving unit operates, the needle assembly 260 combined with the driving shaft 110 may be moved in forward and backward directions (i.e., up and down directions in FIG. 6) of the handpiece 100.

A plurality of RF supply terminals 120 that supplies an RF current generated by the RF generation unit to the micro needle 232 within the treatment tip 200 is provided around the driving shaft 110 exposed to the front end of the handpiece 100.

The plurality of RF supply terminals 120 disposed around the driving shaft 110 is electrically connected to a first substrate 220 of the treatment tip 200 when the treatment tip 200 is mounted on the front end of the handpiece 100. That is, the plurality of RF supply terminals 120 is electrically connected to a plurality of RF input electrodes 225 formed in the first substrate 220 of the treatment tip 200, and transfers an RF current to the needle assembly 260 within the treatment tip 200.

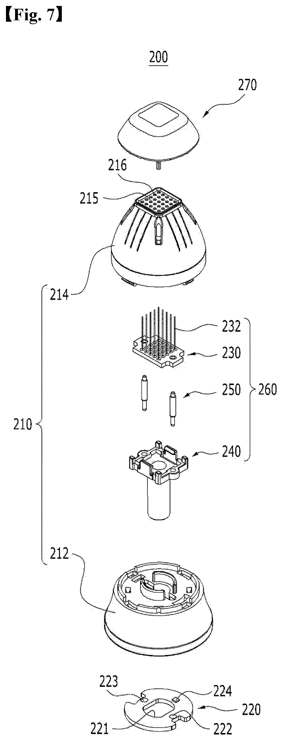

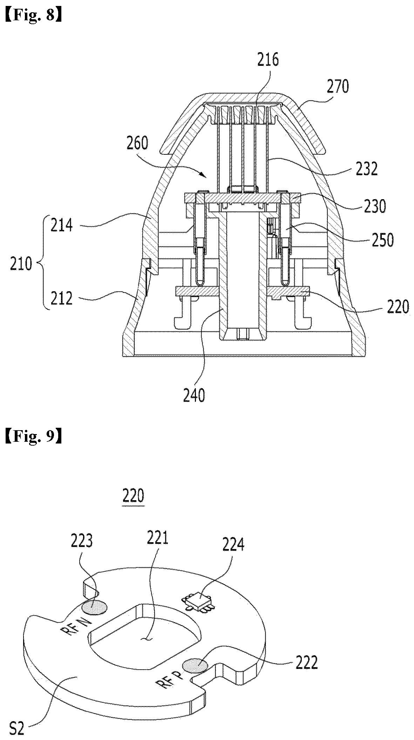

FIG. 7 is a separated perspective view of the treatment tip according to embodiments of the present disclosure. FIG. 8 is a cross-sectional view illustrating a cross-sectional structure of the treatment tip according to embodiments of the present disclosure.

Referring to FIGS. 7 and 8, the treatment tip 200 includes a body 210, the needle assembly 260, and a needle cap 270.

The body 210 has an internal space in which the needle assembly 260 can be accommodated, and is detachably coupled to the front end of the handpiece 100. The driving shaft 110 of the handpiece 100 is connected to the internal space of the body 210. The needle assembly 260 capable of moving in the forward and backward directions (in the up and down directions in the drawings) of the handpiece 100 is disposed in the internal space.

The body 210 includes a first body 212 and a second body 214 having two spit shapes. That is, the body 210 is formed by the mutual coupling of the first body 212 and the second body 214 that are split into two. In this case, the first body 212 and the second body 214 may be detachably coupled. As the body 210 has a structure in which the body can be separated into the two bodies as described above, the needle assembly 260 can be easily installed in the internal space of the body 210.

The first body 212 that forms a lower (rear part) structure of the body 210 is detachably coupled to the front end of the handpiece 100. The first substrate 220 that electrically connects the handpiece 100 and the needle assembly 260 is installed within the first body 212. That is, the first substrate 220 is installed as a structure in which the first substrate 220 has been fixed within the first body 212, and functions to electrically connect the plurality of RF supply terminals 120 exposed to the front end of the handpiece 100 and the needle assembly 260.

The second body 214 that forms an upper (front part) structure of the body 210 is detachably coupled to a front end (top) of the first body 212. The needle assembly 260 that is connected to the driving shaft 110 of the handpiece 100 is disposed within the second body 214. The needle assembly 260 connected to the driving shaft 110 of the handpiece 100 may be moved in the forward and backward directions (in the up and down directions in the drawings) within the second body 214 when the driving shaft 110 operates.

A contact surface 215 that is closely attached to the skin of a procedure subject is formed at a front end of the second body 214. Furthermore, a plurality of needle holes 216 is formed in the contact surface 215 so that the plurality of micro needles 232 provided in the needle assembly 260 can enter the inside of the contact surface 215 and exit the contact surface 215.

Upon procedure using the treatment tip 200, when the needle assembly 260 is advanced through the driving unit of the handpiece 100 in the state in which the skin of the procedure target has been pressed on the contact surface 215 of the second body 214, the micro needle 232 is drawn to the outside of the needle hole 216, and penetrates a skin tissue within the skin. A skin beauty treatment task and a treatment task can be performed by applying an RF to the micro needle 232 through the RF generation unit in this state so that heat energy is transferred to the skin tissue.

The first substrate 220 fixed within the first body 212 functions to electrically connect the handpiece 100 and the needle assembly 260. A first surface S1 of the first substrate 220, which is disposed at the bottom of the first substrate 220 in FIG. 3, is connected to the plurality of RF supply terminals 120 of the handpiece 100. A second surface S2 of the first substrate 220, which is disposed at the top of the first substrate 220 on a side opposite to the first surface S1, is connected to a pair of connection pins 250 of the needle assembly 260. In this case, the first surface S1 of the first substrate 220 refers to a surface in a direction toward the handpiece 100. The second surface S2 refers to a surface in a direction toward the micro needle 232.

The needle assembly 260 includes the plurality of micro needles 232, a second substrate 230 on which the plurality of micro needles 232 is mounted, the holder 240 that supports the second substrate 230, and the pair of connection pins 250 connected to the second substrate 230 through the holder 240. The needle assembly 260 having such a construction is connected to the driving shaft 110 of the handpiece 100, and may be moved in the forward and backward directions within a predetermined stroke range along with the driving shaft 110. Accordingly, the micro needle 232 may protrude to the outside of the second body 214 in the forward and backward directions.

The second substrate 230 on which the plurality of micro needles 232 is mounted is seated at the end of the holder 240 on one side thereof, and is coupled to the holder 240.

The holder 240 supports the second substrate 230 on which the plurality of micro needles 232 is mounted and also enables a connection with the driving shaft 110 of the handpiece 100. That is, a pillar unit of the holder 240 at the center thereof is coupled to the driving shaft 110 of the handpiece 100 through the top of the first body 212 and the first substrate 220.

The pair of connection pins 250 is connected to both opposite sides of the second substrate 230. The pair of connection pins 250 connected to the second substrate 230 is electrically connected to the first substrate 220 through the holder 240. In this case, the end of each of the pair of connection pins 250 may be connected to the first substrate 220 through a closed top of the first body 212. As the pair of connection pins 250 connected to the second substrate 230 as described above electrically connects the first substrate 220 and the second substrate 230, an RF current that is introduced from the handpiece 100 to the first substrate 220 can be introduced into the second substrate 230 through the pair of connection pins 250.

The pair of connection pins 250 that connects the first substrate 220 and the second substrate 230 may have a pogo pin structure having a retractile length. That is, each of the pair of the connection pins 250 is formed to have the pogo pin structure in which a spring is mounted within the connection pin 250, and may be constructed to be retractile in a length direction thereof. As the pair of connection pins 250 is each constructed to have the pogo pin structure in which each connection pin is retractile in the length direction as described above, the second substrate 230 can also maintain an electrical connection state with the first substrate 220 through the pair of connection pins 250 that is retractile although the needle assembly 260 is moved in the forward and backward directions within the second body 214.

Furthermore, the needle cap 270 capable of covering the micro needle 232 so that the micro needle 232 is not exposed to the outside is coupled to the front end of the second body 214. In this case, the needle cap 270 is coupled to the front end of the second body 214 as a structure in which the needle cap 270 is detachable from the front end of the second body 214. Accordingly, a procedure task can be performed in the state in which the needle hole 216 has been exposed to the outside by separating the needle cap 270 from the second body 214.

The treatment tip 200 according to embodiments of the present disclosure is equipped with the NFC chip 224. In the treatment tip 200, the NFC chip 224 is installed at a portion of the first substrate 220, which electrically connects the needle assembly 260 and the handpiece 100.

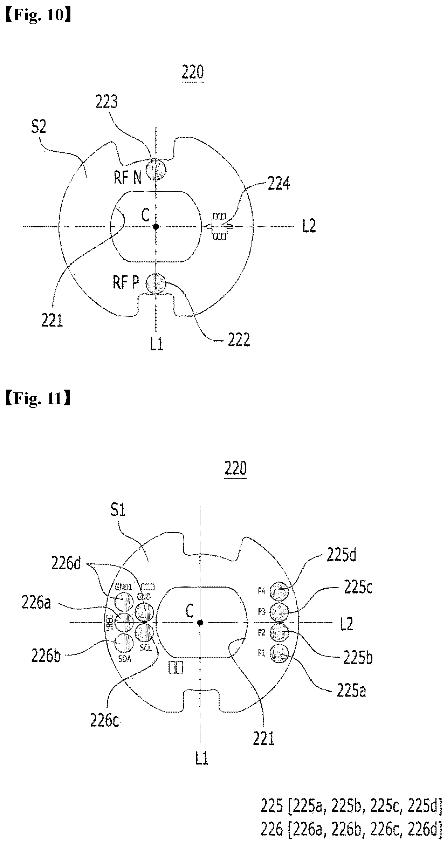

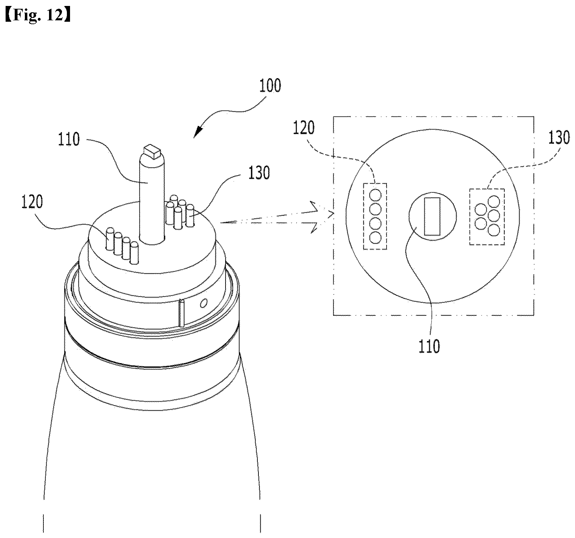

FIG. 9 is a perspective view illustrating a form in which the NFC chip has been installed on the first substrate of the treatment tip. FIGS. 10 and 11 illustrate plan and rear structures of the first substrate illustrated in FIG. 9. Furthermore, FIG. 12 illustrates a structure of the front end of the handpiece in which the plurality of RF supply terminals and signal transmission terminals connected to the plurality of RF input electrodes and signal transmission electrodes formed in the first substrate, respectively, are arranged.

Referring to FIGS. 9 to 12, in the treatment tip 200 according to an embodiment of the present disclosure, the first substrate 220 is fixed and installed in the internal space of the first body 212 that is directly coupled to the handpiece 100.

An opening 221 through which the pillar unit of the holder 240 penetrates is formed at the center of the first substrate 220. Furthermore, the plurality of RF supply terminals 120 of the handpiece 100 is connected to one surface of the first substrate 220, and the pair of connection pins 250 of the needle assembly 260 is connected to the other surface of the first substrate 220.

That is, the plurality of RF input electrodes 225 connected to the plurality of RF supply terminals 120 of the handpiece 100, respectively, is formed on the first surface S1 of the first substrate 220 toward the handpiece 100. A pair of RF output electrodes 222 and 223 that is electrically connected to the plurality of RF input electrodes 225 and to which the pair of connection pins 250 of the needle assembly 260 is connected, respectively, is formed on the second surface S2 on a side opposite to the first surface S1. In this case, the pair of RF output electrodes 222 and 223 formed on the second surface S2 of the first substrate 220 is disposed at positions that are symmetrical to each other with the opening 221 at the center of the first substrate 220 interposed therebetween.

According to an embodiment, the NFC chip 224 may be installed in a part of the first substrate 220 in which a plurality of electrode patterns that electrically connects the pair of connection pins 250 connected to the second substrate 230 and the plurality of RF supply terminals 120 of the handpiece 100 is disposed. The NFC chip 224 installed in the first substrate 220 stores various types of data information related to the specifications of the treatment tip 200, and may perform contactless wireless communication with the external NFC terminal.

Specifically, the NFC chip 224 may be installed in the area of the second surface S2 of the first substrate 220 toward the needle assembly 260. That is, the NFC chip 224 may be installed to be disposed in a surrounding area of the opening 221, which is relatively distant from the pair of RF output electrodes 222 and 223 in the area of the second surface S2 of the first substrate 220.

In this case, the NFC chip 224 disposed in the surrounding area of the opening 221 of the second surface S2 may be disposed at a location around the opening 221 through which a virtual second straight line L2 that vertically intersects the center C of a virtual first straight line L1 that connects the pair of RF output electrodes 222 and 223 passes (refer to FIG. 10).

If the NFC chip 224 is disposed to have such a structure, intervals between the NFC chip 224 and the pair of RF output electrodes 222 and 223 on both sides of the NFC chip 224 can be identically maintained, and the NFC chip 224 can be disposed at a location as far away as possible from the pair of RF output electrodes 222 and 223 on both sides of the NFC chip 224. Accordingly, a reduction of NFC communication performance, which occurs due to electromagnetic interference between the NFC chip 224 and the pair of RF output electrodes 222 and 223 on both sides of the NFC chip 224 can be minimized, and reliability of communication performance can be improved.

An electrode group 226 for signal transmission that is connected to the NFC chip 224 mounted on the second surface S2 in the form of a circuit pattern, that is, a DC power electrode 226a, electrodes 226b and 226c for data communication, and a ground electrode 226d, are arranged to form a group in one area around the opening 221 on the first surface S1 of the first substrate 220.

In accordance with the electrode group 226, a plurality of terminals 130 for signal transmission, which is connected to the DC power electrode 226a, electrodes 226b and 226c for data communication, and ground electrode 226d of the first substrate 220, is arranged to form a group in the same array form around the driving shaft 110 of the handpiece 100.

The DC power electrode 226a formed in the first substrate 220 functions as a power line that supplies power to the NFC chip 224. Accordingly, the NFC chip 224 may perform short distance communication with the external NFC terminal by being supplied with power from the handpiece 100 through the DC power electrode 226a.

Furthermore, the electrodes 226b and 226c for data communication function as signal lines for communication with the handpiece 100. The electrodes 226b and 226c for data communication include serial data (SDA) 226b and a serial clock (SCL) 226c. The SDA 226b and the SCL 226c are directly connected to the control unit (main controller) of the handpiece 100, and may exchange data with the control unit.

In this case, the SDA 226b functions as a signal line for the transmission and reception of data. The NFC chip 224 may transmit and receive data to and from the control unit of the handpiece 100 through the SDA 226b. Furthermore, the SCL 226c functions as a signal line that synchronizes a data transfer rate, and provides timing while data are transmitted by transmitting a signal at a predetermined cycle when the data are transmitted. Furthermore, the ground electrode 226d stabilizes a change in the voltage or current, maintains the stability of a circuit by removing noise generated within the circuit, and improves performance.

The DC power electrode 226a, the electrodes 226b and 226c for data communication, and the ground electrode 226d form the electrode group 226 for signal transmission in which the DC power electrode 226a, the electrodes 226b and 226c for data communication, and the ground electrode 226d are arranged to form a group on the first surface S1 of the first substrate 220 disposed on the side opposite to the NFC chip 224.

Furthermore, as described above, the plurality of RF input electrode 225 connected to the plurality of RF supply terminals 120 exposed to the front end of the handpiece 100, respectively, is formed on the first surface S1 of the first substrate 220. In this case, four RF input electrode 225 may be arranged and formed in a row in parallel to the virtual first straight line L1 in the surrounding area of the opening 221.

In this case, first and second RF input electrodes 225a and 225b, among the four RF input electrodes 225a to 225d, may be connected to the first RF output electrode 222 disposed on the second surface S2. Third and fourth RF input electrodes 225c and 225d, among the four RF input electrodes 225a to 225d, may be connected to the second RF output electrode 223 disposed on the second surface S2. In this case, the first RF output electrode 222 is an electrode from which an RF signal (+) is output, and the second RF output electrode 223 is an electrode from which an RF signal (-) is output.

The plurality of RF input electrodes 225 may be disposed at locations at which the plurality of RF input electrodes 225 faces the DC power electrode 226a, the electrodes 226b and 226c for data communication, and the ground electrode 226d that are arranged to form a group with the opening 221 at the center C of the opening 221 interposed therebetween (refer to FIG. 11).

That is, the electrode group, including the DC power electrode 226a, the electrodes 226b and 226c for data communication, and the ground electrode 226d, and the four RF input electrodes 225a to 225d may be disposed at the locations at which the electrode group and the four RF input electrodes 225a to 225d face each other in a direction toward the virtual second straight line L2 that passes through the center C of the opening 221.

When the electrode group and the four RF input electrodes 225a to 225d are disposed as described above, the plurality of RF input electrodes 225a to 225d through which an RF current flows and the four electrodes 226a to 226d for signal transmission that form a group in the first substrate 220 are disposed at the locations as far away as possible. Accordingly, a reduction of communication performance attributable to electromagnetic interference between the electrodes disposed on both sides can be prevented, and reliability of communication performance can be improved.

In contrast, if the NFC chip 224 is constructed to operate by wirelessly receiving power from the external NFC terminal without receiving power from the handpiece 100, power consumption of the NFC chip 224 may be increased due to electromagnetic interference between the electrodes on both sides. Accordingly, a power efficiency reduction problem of the NFC chip 224, which occurs because the NFC chip 224 consumes additional power due to electromagnetic interference between the electrodes on both sides through such a construction, can be prevented. The coverage reduction and data transmission instability of the NFC chip, which occur because an NFC signal is attenuated due to electromagnetic interference, can also be overcome.

Furthermore, an additional instrument or wire for the installation of the NFC chip 224 is not required because the NFC chip 224 is installed in a part of the first substrate 220 in which various electrode patterns that connect the handpiece 100 and the pair of connection pins 250 are disposed. A device structure within the treatment tip 200 does not become complex and costs according to the installation of the NFC chip 224 can also be reduced because the NFC chip 224 can be simply installed by changing only a circuit pattern structure of the first substrate 220.

In particular, the NFC chip 224 is installed around the opening 221 on the second surface S2 of the first substrate 220, but is installed at the location through which the virtual second straight line L2 that vertically intersects the center C of the virtual first straight line L1 that connects the pair of RF output electrodes 222 and 223 on both sides of the center C passes. The distances between the NFC chip 224 and the pair of RF output electrodes 222 and 223 on both sides of the center C are spaced apart from each other as much as possible. Accordingly, a communication error problem that occurs due to electromagnetic interference between the NFC chip 224 and the pair of RF output electrodes 222 and 223 can be prevented, and NFC communication performance and reliability can be improved.

Furthermore, the DC power electrode 226a, the electrodes 226b and 226c for data communication, and the ground electrode 226d that are arranged to form a group in a way to be circuit-connected to the NFC chip 224 on the first surface S1 of the first substrate 220 are disposed at the locations at which the DC power electrode 226a, the electrodes 226b and 226c for data communication, and the ground electrode 226d are symmetrical to the plurality of RF input electrodes 225 with the opening 221 interposed therebetween. Accordingly, a reduction of communication performance attributable to electromagnetic interference can be prevented and reliability of NFC communication performance can be improved because a separation distance between the plurality of RF input electrodes 225 and the electrodes 226a to 226d for signal transmission through which an RF current flows can be maximized.

Furthermore, if the NFC chip 224 is constructed to operate by directly receiving power from the external NFC terminal wirelessly, a power efficiency reduction problem of the NFC chip 224, which occurs because the NFC chip 224 consumes additional power due to electromagnetic interference between the electrodes on both sides of the NFC chip 224, can be overcome. Coverage reduction and data transmission instability problems of the NFC chip 224, which occur because an NFC signal is attenuated due to electromagnetic interference, can also be overcome.

Furthermore, the body 210 of the treatment tip 200 is constructed to be separated into the first body 212 and the second body 214 which are mutually detachable. Accordingly, the needle assembly 260 can be further easily installed within the second body 214 because the second body 214 is separated from the first body 212. If NFC communication is not smooth, after the second body 214 is separated from the first body 212, the external NFC terminal may be made closest to the NFC chip 224 installed in the first body 212 for smooth NFC communication.

Although the embodiments of the present disclosure have been described above, the scope of the present disclosure is not limited to only such specific embodiments, and a person having ordinary knowledge in a corresponding field may properly change the present disclosure without departing from the category of the claims of the present disclosure.

## Claims

1. A dermatologic treatment device performing a skin procedure operation, the dermatologic treatment device comprising:
a treatment tip configured to come into contact with a skin of a patient and to deliver energy; and
an applicator connected to the treatment tip,
wherein the treatment tip comprises a near field communication (NFC) chip configured to store treatment tip data, and
wherein the applicator is configured to read the treatment tip data from the NFC chip and to perform the skin procedure operation based on the treatment tip data.

2. The dermatologic treatment device of claim 1, wherein the applicator detects a connection of the treatment tip and the applicator and transmits a command to request the reading of the treatment tip data to the NFC chip when the treatment tip is connected to the applicator.

3. The dermatologic treatment device of claim 1, wherein:
the treatment tip data comprise identification data for identifying whether the treatment tip is able to be used normally, and
the applicator
receives the identification data from the NFC chip,
compares the identification data and reference identification data stored in the applicator,
operates in an operation mode when the identification data are stored in the reference identification data, and
operates in a non-operation mode when the identification data are not included in the reference identification data.

4. The dermatologic treatment device of claim 3, wherein the identification data indicate at least one of a country in which the treatment tip is used, whether the treatment tip is a genuine product, and a serial number of the treatment tip.

5. The dermatologic treatment device of claim 1, wherein:
the treatment tip data comprise count data indicative of a number of uses of the treatment tip, and
the applicator
receives the count data from the NFC chip,
compares the count data and reference count data stored in the applicator,
operates in an operation mode when the count data are less than the reference count data, and
operates in a non-operation mode when the count data are equal to or greater than the reference count data.

6. The dermatologic treatment device of claim 5, wherein the applicator transmits a value indicative of the number of uses of the treatment tip according to a skin procedure of the dermatologic treatment device to the NFC chip after operating in the operation mode.

7. The dermatologic treatment device of claim 1, wherein:
the treatment tip data comprise operation data indicative of an operation environment of the treatment tip, and
the applicator
receives the operation data from the NFC chip,
determine an operation variable corresponding to an operation environment indicated by the operation data,
stores the determined operation variable as a setting value, and
performs the skin procedure operation based on the stored setting value.

8. The dermatologic treatment device of claim 7, wherein:
the operation data indicate characteristics of radio frequency (RF) power of the skin procedure operation,
the applicator
determines the operation variable indicative of the characteristics the RF power based on the operation data,
stores the determined operation variable as the setting value, and
outputs RF power for the skin procedure operation based on the setting value.

9. The dermatologic treatment device of claim 7, wherein:
the operation data are data related to a movement of the treatment tip,
the applicator
determines an operation variable indicative of a movement of the treatment tip based on the operation data and stores the operation variable as the setting value, and
moves the treatment tip based on the setting value.

10. An operating method of a dermatologic treatment device comprising a treatment tip including a near field communication (NFC) chip, the operating method comprising:
detecting a connection of the treatment tip;
reading treatment tip data from the NFC chip of the connected treatment tip when the treatment tip is connected; and
performing a skin procedure operation based on the treatment tip data.

11. The operating method of claim 10, wherein:
the treatment tip data comprise identification data for identifying whether the treatment tip is able to be used normally, and
the performing of the skin procedure operation comprises:
receiving the identification data from the NFC chip,
comparing the identification data and reference identification data stored in an applicator,
operating in an operation mode when the identification data are stored in the reference identification data, and
operating in a non-operation mode when the identification data are not included in the reference identification data.

12. The operating method of claim 11, wherein the identification data indicate at least one of a country in which the treatment tip is used, whether the treatment tip is a genuine product, and a serial number of the treatment tip.

13. The operating method of claim 10, wherein:
the treatment tip data comprise count data indicative of a number of uses of the treatment tip, and
the performing of the skin procedure operation comprises:
receiving the count data from the NFC chip,
comparing the count data and reference count data stored in the applicator,
operates in the operation mode when the count data are less than the reference count data, and
operating in the non-operation mode when the count data are equal to or greater than the reference count data.

14. The operating method of claim 13, wherein the performing of the skin procedure operation comprises transmitting a value indicative of the number of uses of the treatment tip according to a skin procedure of the dermatologic treatment device to the NFC chip after operating in the operation mode.

15. The operating method of claim 10, wherein
the treatment tip data comprise operation data indicative of an operation environment of the treatment tip, and
the performing of the skin procedure operation comprises:
receiving the operation data from the NFC chip,
determining an operation variable corresponding to an operation environment indicated by the operation data,
storing the determined operation variable as a setting value, and
performing the skin procedure operation based on the stored setting value.
